# EUROPEAN PATENT APPLICATION

(11) **EP 4 545 106 A1**
(43) Date of publication of application: **30.04.2025**
(21) Application number: 23827248.8
(22) Date of filing: 21.06.2023
(51) Int. Cl.: A61L 27/18, A61L 27/20, A61L 27/44, A61L 27/58, C12M 3/00, C12N 5/074

(54) **TISSUE REGENERATION PROMOTION SHEET**

(30) Priority: 22.06.2022 JP 2022100731; 30.03.2023 JP 2023054932
(71) Applicant: Fujita Academy, Toyoake-shi, Aichi 470-1192 (JP)
(72) Inventor: HORI Hideo, Toyoake-shi, Aichi 470-1192 (JP); FUJITA Nobuyuki, Toyoake-shi, Aichi 470-1192 (JP); KAWABATA Soya, Toyoake-shi, Aichi 470-1192 (JP)
(74) Representative: Heyerhoff Geiger GmbH & Co. KG
(86) International application number: PCT/JP2023/022993
(87) International publication number: WO 2023/249065

(57) **Abstract**

A tissue regeneration promoting sheet (10) includes a cell retention sheet (12) that is a nonwoven fabric, and the nonwoven fabric contains fibers based on polycaprolactone as a main component and is configured to be impregnated with a cell suspension solution containing stem cells. The fiber diameter of the fibers forming the nonwoven fabric may be in a range of 300 nm to 2 µm. The basis weight of the nonwoven fabric may be in a range of 1 g/m² to 3 g/m². The thickness of the nonwoven fabric may be larger than or equal to 300 µm. The tissue regeneration promoting sheet (10) may further include a reinforcement sheet (14) laminated on the nonwoven fabric and configured to be impregnated with the cell suspension solution.

## Description

### [Technical Field]

The present invention relates to a tissue regeneration promoting sheet.

### [Background Art]

In recent years, in the field of regenerative medicine, there has been progress in the development of tissue regeneration promoting materials that can retain cells therein and promote tissue regeneration by being embedded in the body. For example, Patent Literature 1 discloses a sheet-like tissue regeneration promoting material in which at least one of the groups consisting of leukocytes and platelets is present on the surface thereof. Further, Patent Literature 2 discloses a liquid tissue regeneration promoting material obtained by mixing polymer material powder with cells.

### [Citation List]

### [Patent Literature]

Patent Literature 1: Japanese Patent No. 4847129
Patent Literature 2: Japanese Patent Application Laid-Open No. 2015-112262

### [Summary of Invention]

### [Technical Problem]

However, since the tissue regeneration promoting materials disclosed in Patent Literatures 1 and 2 use porous bodies or polymer material powder, there is a problem of difficulty in stably retaining mesenchymal stem cells having a high tissue regenerative capacity among various cells. Such a problem exists not only when mesenchymal stem cells are retained by tissue regeneration promoting materials but also when other stem cells are retained.

The present invention has been made based on such a background, and an object of the present invention is to provide a tissue regeneration promoting sheet that can stably retain stem cells.

### [Solution to Problem]

To achieve the object described above, a tissue regeneration promoting sheet according to the present invention includes a nonwoven fabric, the nonwoven fabric containing fibers based on polycaprolactone as a main component, and the nonwoven fabric being configured to be impregnated with a cell suspension solution containing stem cells.

The fiber diameter of the fibers forming the nonwoven fabric may be in a range of 300 nm to 2 µm.

The basis weight of the nonwoven fabric may be in a range of 1 g/m² to 3 g/m².

The thickness of the nonwoven fabric may be larger than or equal to 300 µm.

The tissue regeneration promoting sheet may further include a reinforcement sheet laminated on the nonwoven fabric and configured to be impregnated with the cell suspension solution.

The reinforcement sheet may be another nonwoven fabric formed of fibers that are thicker than fibers forming the nonwoven fabric.

The nonwoven fabric may include a first nonwoven fabric and a second nonwoven fabric, the second nonwoven fabric being laminated on the first nonwoven fabric and being less likely to be deformed than the first nonwoven fabric.

### [Advantageous Effects of Invention]

According to the present invention, it is possible to provide a tissue regeneration promoting sheet that can stably retain stem cells.

### [Brief Description of Drawings]

[FIG. 1]

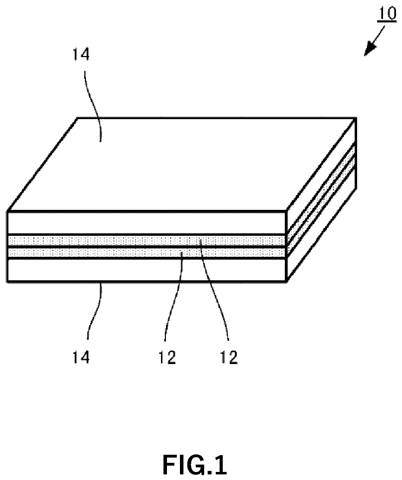

   FIG. 1 is a perspective view illustrating a configuration of a tissue regeneration promoting sheet according to an embodiment of the present invention.
[FIG. 2]

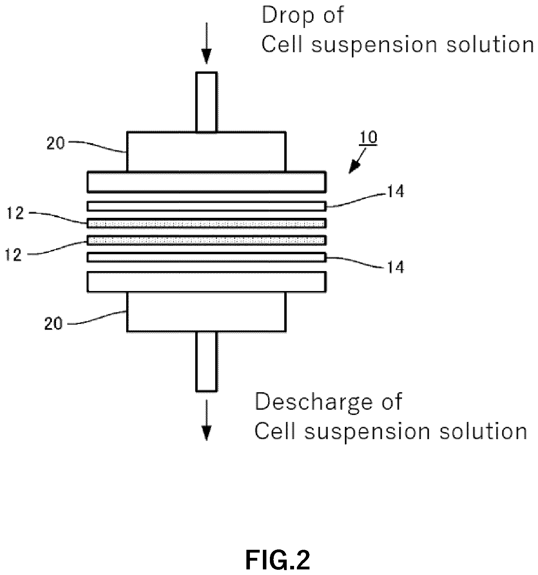

   FIG. 2 is a front view illustrating an overview of producing the tissue regeneration promoting sheet according to the embodiment of the present invention.
[FIG. 3]

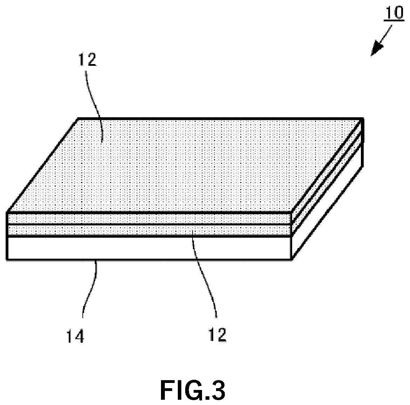

   FIG. 3 is a perspective view illustrating a configuration of a tissue regeneration promoting sheet according to a modified example of the present invention.
[FIG. 4]

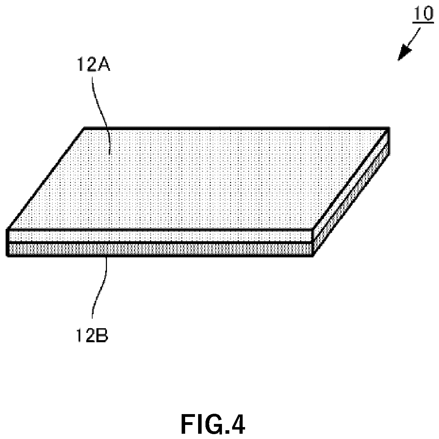

   FIG. 4 is a perspective view illustrating a configuration of a tissue regeneration promoting sheet according to another modified example of the present invention.
[FIG. 5]

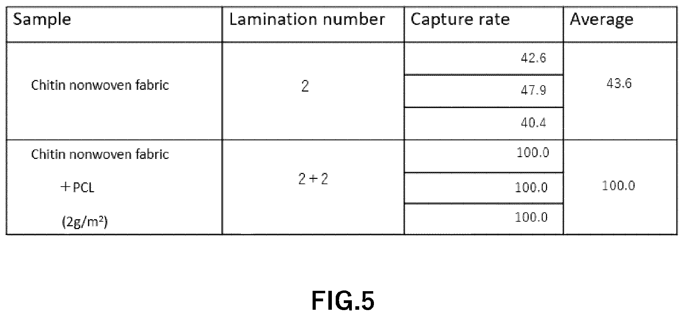

   FIG. 5 is a diagram illustrating the capture rate of mesenchymal stem cells with respective samples in Example 1.
[FIG. 6]

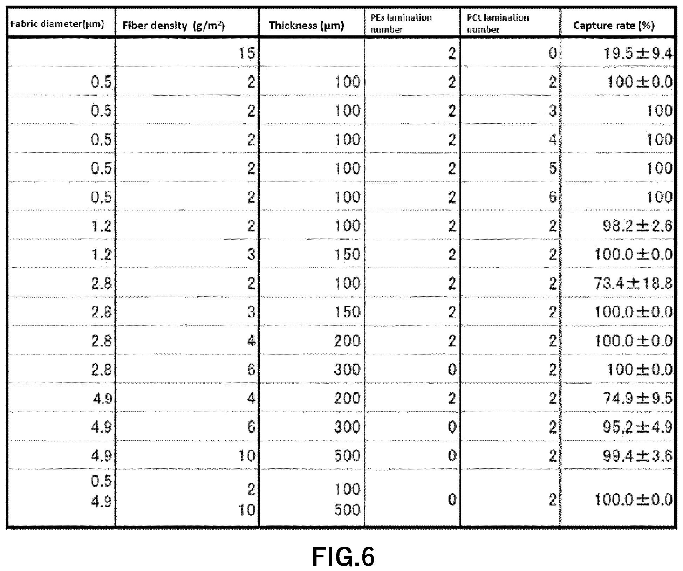

   FIG. 6 is a diagram illustrating the capture rate of mesenchymal stem cells with respective samples in Example 2.
[FIG. 7]

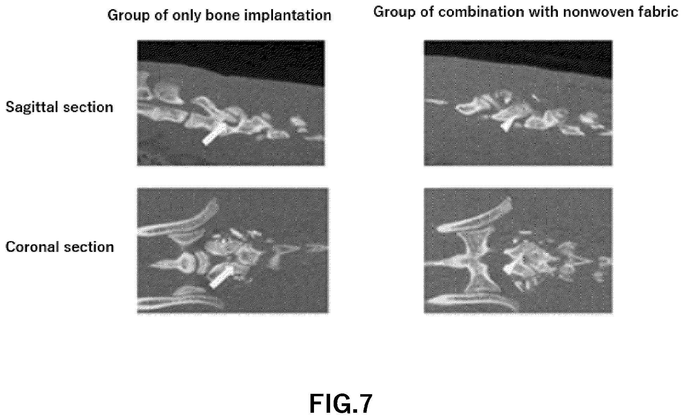

   FIG. 7 is a diagram illustrating CT images of rat cervical vertebrae in Example 3.
[FIG. 8]

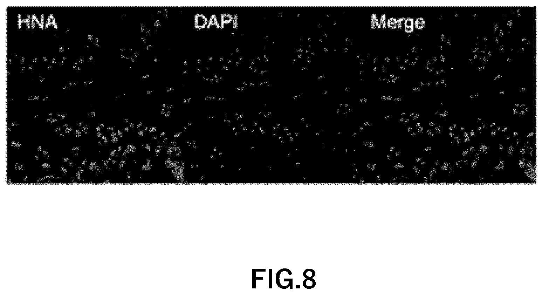

   FIG. 8 is a diagram illustrating immunostaining images at six weeks after bone implantation in Example 3.

### [Description of Embodiments]

A tissue regeneration promoting sheet according to an embodiment of the present invention will be described below in detail with reference to the drawings. In the drawings, the same or equivalent parts are labeled with the same reference numerals.

The tissue regeneration promoting sheet according to the embodiment is a sheet that promotes tissue regeneration at a damaged site by being embedded in the damaged site in a body. The tissue regeneration promoting sheet according to the embodiment includes a nonwoven fabric formed of small-diameter fibers, for example, microfibers, to which stem cells adhere. The nonwoven fabric is a sheet in which small-diameter fibers are intertwined without being woven. The nonwoven fabric can be deformed in any manner and thus is suitable for being embedded in or attached to a damaged site in accordance with the shape of the damaged site. The tissue regeneration promoting sheet can be attached to or embedded in any organs, for example, hearts, blood vessels, lungs, trachea, esophagus, stomachs, small intestines, large intestines, livers, kidneys, bladders, bones, muscles, or skins. The tissue regeneration promoting sheet can be caused to function as a scaffold that promotes proliferation of specific cells in the body.

As illustrated in FIG. 1, the tissue regeneration promoting sheet 10 includes cell retention sheets 12 that retain stem cells and a pair of reinforcement sheets 14 that are bonded so as to interpose the cell retention sheets 12 from above and below to reinforce the cell retention sheets 12. The reason why the cell retention sheets 12 are interposed between and reinforced by the pair of reinforcement sheets 14 is that the nonwoven fabric forming the cell retention sheet 12 tends to easily curl up when being wet due to properties of the raw material described below. The cell retention sheet 12 and the reinforcement sheet 14 may be bonded to each other via an adhesive agent or heat fusion or may be bonded by intertwining of fibers during production of the nonwoven fabric.

The number of cell retention sheets 12 arranged between the pair of reinforcement sheets 14 may be any number as long as a cell suspension solution containing stem cells can be filtrated. However, to prevent the cell suspension solution from leaking from holders described later when filtrating the cell suspension solution, the number of laminations of the cell retention sheets 12 is preferably two or greater. The cell suspension solution is a solution in which cells are suspended in a liquid that can be administered into a body. When a plurality of cell retention sheets 12 are laminated on each other, the plurality of cell retention sheets 12 may be bonded to each other by the same scheme as used when the cell retention sheet 12 and the reinforcement sheet 14 are laminated on each other. Note that, in FIG. 1, to facilitate understanding, the thickness direction of the tissue regeneration promoting sheet 10 is depicted in exaggeration. The thickness of the tissue regeneration promoting sheet 10 is set to the extent that enables filtration of a cell suspension solution.

The cell retention sheet 12 is a nonwoven fabric containing fibers based on polycaprolactone as the main component. As a result of intensive study by the inventors, it has been found that nonwoven fabrics containing fibers based on polycaprolactone as the main component makes it possible to capture stem cells with high efficiency when a cell suspension solution containing stem cells is filtrated and that the activity in adhered stem cells is promoted. Polycaprolactone is a semicrystalline and biodegradable thermoplastic polyester, which is also suitable for production of nonwoven fabrics. For example, Polycaprolactone is poly-ε-caprolactone. The molecular weight of polycaprolactone may be a molecular weight suitable for injection molding, which is in a range of 70,000 to 100,000, for example.

The fibers based on polycaprolactone as the main component may be formed of only polycaprolactone or may contain a further substance. As the further substance, for example, agents that promote tissue regeneration or a further biodegradable polymer may be used. The agents that promote tissue regeneration may be, for example, hydroxyapatite (calcium phosphate hydroxide) or tricalcium phosphate. The further biodegradable polymer may be, for example, polylactic acid, polyglycolic acid, polydioxanone, or copolymers thereof.

With a smaller fiber diameter, the fibers forming the cell retention sheet 12 will be improved in the capture rate of stem cells but will tend to curl up easily when the cell retention sheet 12 absorbs water. The fiber diameter of the fibers forming the cell retention sheet 12 is in a range of 100 nm to 3 µm, for example, and preferably in a range of 500 nm to 1 µm in view of the capture rate of stem cells and ease of handling the cell retention sheet 12.

In the cell retention sheet 12, a larger basis weight thereof makes it possible to increase the amount of adhered stem cells but gradually makes it difficult to filtrate the cell suspension solution. The basis weight of the cell retention sheet 12 is in a range of 1 g/m² to 3 g/m², for example, preferably in a range of 1.5 g/m² to 2.5 g/m², and more preferably 2 g/m² in view of the amount of adhered stem cells and ease of production of the cell retention sheet 12.

For example, the thickness of the cell retention sheet 12 is, preferably, larger than or equal to 300 µm, and, more preferably, larger than or equal to 500 µm in view of ease of handling the cell retention sheet 12 with tweezers. However, it should be noted that a larger thickness of the cell retention sheet 12 makes it more difficult to filtrate the cell suspension solution.

The stem cells to be adhered to the cell retention sheet 12 are preferably mesenchymal stem cells (MSC) or cells differentiated from mesenchymal stem cells. The mesenchymal stem cells are somatic stem cells having ability of differentiation into cells belonging to the mesenchyme and may be any of those derived from fat, periosteum, synovium, cancellous bone, bone marrow, amniotic membrane, cord blood, and placenta. Further, the stem cells to be adhered to the nonwoven fabric may be, for example, embryonic stem (ES) cells or induced pluripotent stem (iPS) cells. Multiple types of stem cells may be adhered to the nonwoven fabric together.

The stem cells to be adhered to the cell retention sheet 12 may be collected from a patient and then cultured. To culture stem cells in vitro, for example, a culture medium containing fetal bovine serum (FBS) or human serum, preferably patient-derived human serum or a serum-free culture medium may be used. The stem cells cultured in a culture medium may be directly adhered to the nonwoven fabric, or the stem cells may be differentiated to express specific cells, for example, osteoblasts, which may then be adhered to the nonwoven fabric. To express specific cells, for example, a differentiation-inducing factor may be added to a culture medium in which the stem cells are cultured.

Note that the stem cells are not necessarily required to be adhered in advance to the cell retention sheet 12, and the cell retention sheet 12 may be allowed to capture the stem cells in a body by administering a cell suspension solution containing stem cells to vasculature after arranging the tissue regeneration promoting sheet 10 at a damaged site in the body.

The reinforcement sheet 14 is a nonwoven fabric that contains fibers based on a biocompatible polymer as the main component and can cause the cell suspension solution to pass therethrough. The reinforcement sheet 14 is bonded to the cell retention sheet 12 in order to reinforce the cell retention sheet 12, which tends to curl up and easily deform. The biocompatible polymer forming the reinforcement sheet 14 is a biocompatible polymer having greater strength than polycaprolactone, for example, a biodegradable polymer such as chitin, chitosan, polylactic acid, or polyglycolic acid, and among these biodegradable polymers, chitin is preferable. The fiber diameter of the microfibers in the reinforcement sheet 14 is preferably larger than the fiber diameter of the microfibers in the cell retention sheet 12. Note that, although stem cells are not required to be attached to the reinforcement sheet 14, the stem cells may be adhered to the reinforcement sheet 14 in a process of adhering the stem cells to the cell retention sheet 12.

The above is the configuration of the tissue regeneration promoting sheet 10.

Next, the specific procedure to adhere stem cells to the cell retention sheet 12 according to the embodiment will be described. To adhere stem cells to the cell retention sheet 12, it is sufficient to allow the cell retention sheet 12 to be in contact with a cell suspension solution containing the stem cells for a while. However, to suppress a reduction in the growth factor production capacity during culture of stem cells, it is preferable to perform the following procedure.

First, as illustrated in FIG. 2, the cell retention sheets 12 and the reinforcement sheets 14 laminated on each other are set between a pair of holders 20 forming a filtration device. Each holder 20 is configured so that the cell suspension solution can flow out of and flow into the holder 20, and the cell suspension solution comes into contact with the cell retention sheet 12 when the cell suspension solution is caused to flow from one holder 20 to the other holder 20. Note that, although there is a gap between these components in FIG. 2, the two cell retention sheets 12 bonded to each other are interposed and bonded between the pair of reinforcement sheets 14, and in this state, the pair of holders 20 are in close contact with each other in the actual practice.

Next, the cell suspension solution containing stem cells is slowly dropped drop by drop from above, and the cell suspension solution is filtrated through the two cell retention sheets 12. The filtration rate is, for example, a rate at which 1 ml of the cell suspension solution is dropped over 2 to 3 minutes. The stem cells contained in the cell suspension solution dropped at this time adhere by themselves to the fibers of the cell retention sheet 12. Although the exact mechanism is unknown, when the cell suspension solution containing stem cells is filtrated little by little through a nonwoven fabric formed of fibers based on polycaprolactone as the main component, the stem cells stably adhere to each fiber, the adhered stem cells are activated, and production of growth factors is promoted.

The above is the specific procedure to adhere stem cells to the cell retention sheet 12.

Note that, as long as the stem cells are adhered to the cell retention sheet 12, production of growth factors can be more or less expected from stem cells, and therefore the above procedure is not necessarily required to be used.

As described above, the tissue regeneration promoting sheet 10 according to the embodiment includes the cell retention sheet 12, which contains fibers based on polycaprolactone as the main component and can be impregnated with a cell suspension solution containing stem cells, and the reinforcement sheet 14, which is laminated on the cell retention sheet 12 and can be impregnated with the cell suspension solution. Thus, the cell retention sheet 12 can stably retain stem cells and improve the ease of handling the cell retention sheet 12 at the user.

The present invention is not limited to the embodiment described above, and modification described below is also possible.

### [Modified Example]

Although two cell retention sheets 12 are arranged between the pair of reinforcement sheets 14 in the embodiment described above, the present invention is not limited thereto. The number of cell retention sheets 12 interposed between the pair of reinforcement sheets 14 may be one or three or greater.

Although the reinforcement sheet 14 is a nonwoven fabric in the embodiment described above, the present invention is not limited thereto. For example, the reinforcement sheet 14 may be a porous body that can be impregnated with the cell suspension solution.

Although the reinforcement sheets 14 interpose the cell retention sheets 12 from above and below in the embodiment described above, the present invention is not limited thereto. For example, as illustrated in FIG. 3, the reinforcement sheet 14 may be laminated on one side of the cell retention sheet 12 to form the tissue regeneration promoting sheet 10. When the strength of the tissue regeneration promoting sheet 10 can be ensured even when the reinforcement sheet 14 is omitted, or when the strength of the tissue regeneration promoting sheet 10 is not required to be ensured, the reinforcement sheet 14 may be omitted. The case where the strength of the tissue regeneration promoting sheet 10 is not required to be ensured may be, for example, a case where the tissue regeneration promoting sheet 10 is accommodated in a space in an implant or a case where the tissue regeneration promoting sheet 10 is curled up and embedded in a damaged site in the body.

To ensure the strength of the tissue regeneration promoting sheet 10 even when the reinforcement sheet 14 is omitted, for example, at least one of the fiber diameter of the fibers forming the cell retention sheet 12, the fiber density of the same, and the thickness of the cell retention sheet 12 can be increased. Further, as illustrated in FIG. 4, the tissue regeneration promoting sheet 10 may be formed by laminating a first cell retention sheet 12A and a second cell retention sheet 12B, which is less likely to be deformed than the first cell retention sheet 12A, on each other. The second cell retention sheet 12B may have a larger fiber diameter or a higher fiber density than the first cell retention sheet 12A, for example. The first cell retention sheet 12A is an example of the first nonwoven fabric, and the second cell retention sheet 12B is an example of the second nonwoven fabric that is less likely to be deformed than the first nonwoven fabric.

The embodiment described above is an example, the present invention is not limited thereto, and various embodiment are possible without departing from the spirit of the invention recited in the claims. The components described in the embodiment and modified examples can be combined in any manner. Furthermore, the present invention includes inventions equivalent to the inventions recited in the claims.

Examples will be provided below to specifically describe the present invention. However, the present invention is not limited to these Examples.

### EXAMPLES

### [Example 1]

In Example 1, sheets with lamination of a polycaprolactone (PCL) nonwoven fabric and a chitin nonwoven fabric were prepared by using the scheme according to the above embodiment, and the capture rates of mesenchymal stem cells with respective sheets were evaluated. To be specific, first, as illustrated in FIG. 1, two pieces of PCL nonwoven fabric were interposed between one piece of chitin nonwoven fabric above and one piece of chitin nonwoven fabric below and were then bonded together. The PCL nonwoven fabric has a fabric diameter of 0.5 to 1 µm and a fiber density of 2 g/m², and the chitin nonwoven fabric has a fabric diameter of 8 µm and a fiber density of 30 g/m². Next, mesenchymal stem cells were adhered to the PCL nonwoven fabric by passing a cell suspension solution containing human mesenchymal stem cells through the sheets interposed between holders used for filtration sterilization, and then the capture rate of mesenchymal stem cells was evaluated. Swinnex filter holder (Merck Millipore) was used as the holders. As comparative examples, similar experiments were performed, respectively, on sheets with lamination of only two pieces of chitin nonwoven fabric. The experiments were performed on three samples for each sheet.

FIG. 5 illustrates the results. The average cell capture rate was 43.6% for the sheet with lamination of only the chitin nonwoven fabric, whereas the average cell capture rate was 100% for the sheet with lamination of the PCL nonwoven fabric and the chitin nonwoven fabric. From the above, it was confirmed that the PCL nonwoven fabric allows stem cells to be adhered with high efficiency.

### [Example 2]

In Example 2, a sheet with lamination of a PCL nonwoven fabric and a polyester (PEs) nonwoven fabric, a sheet of a PCL nonwoven fabric alone, and a sheet with lamination of two types of PCL nonwoven fabrics having different characteristics were prepared, and ease of handling the sheets was examined. Further, a cell suspension solution was dropped onto each sample, and the cell capture rate was measured.

An attempt to set the sheet of the PCL nonwoven fabric alone between the holders revealed that, when the thickness of the PCL nonwoven fabric was 200 µm or less, the PCL nonwoven fabric alone was unable to be set between the holders. Accordingly, when the thickness of the PCL nonwoven fabric was 200 µm or less, the PCL nonwoven fabric and the PEs nonwoven fabric were laminated, and when the thickness of the PCL nonwoven fabric was 300 µm or greater, a sheet of the PCL nonwoven fabric alone was set between the holders. Further, a sheet with lamination of a PCL nonwoven fabric having a fiber diameter of 0.5 µm, a fiber density of 2 g/m², and a thickness of 100 µm and a PCL nonwoven fabric having a fiber diameter of 4.9 µm, a fiber density of 10 g/m², and a thickness of 500 µm was prepared, and this sheet was able to be set between the holders. Other procedures were the same as those in Example 1. While the experiments were performed with three samples for each condition, the experiments were performed with only one sample for each sheet having a fiber diameter of 0.5 µm, a fiber density of 2 g/m², a thickness of 100 µm, a PEs lamination number of 2, and a PCL lamination number of 3 to 6.

FIG. 6 illustrates the results. Focusing on the data group of the PEs lamination number of 2, the PCL lamination number of 2, and the thickness of 200 µm, it was found that the smaller the fiber diameter was, the higher the cell capture rate was. To be specific, when the fiber diameter was 0.5 µm, the cell capture rate was 100±0.0%, whereas when the fiber diameter was 2.8 µm, the cell capture rate was 73.4±18.8%. Further, focusing on the data group of a fiber diameter of 4.9 µm, it was found that the larger the thickness was, the higher the cell capture rate was. To be specific, when the thickness was 200 µm, the cell capture rate was 74.9±9.5%, whereas when the thickness was 500 µm, the cell capture rate was 99.4±3.6%. From the above, it was confirmed that a smaller fiber diameter and a larger thickness result in a higher capture rate of mesenchymal stem cells.

### [Example 3]

In Example 3, a sheet with lamination of a PCL nonwoven fabric and a chitin nonwoven fabric was implanted together with bone fragments into the cervical vertebrae of three immunodeficient model rats, and bone healing was manually evaluated at six weeks after the implantation. Further, the evaluation of bone healing was also performed by using computed tomography (CT) images of the cervical vertebrae of immunodeficient model rats and immunostaining images of collected tissue sections. Immunostaining images were obtained by using a fluorescence microscope to take tissue sections stained with DAPI (4',6-diamidino-2-phenylindole), which is a fluorescent dye that binds to DNA (deoxyribonucleic acid). As the sheet with lamination of the PCL nonwoven fabric and the chitin nonwoven fabric, a sheet to which human mesenchymal stem cells were adhered was used. As a comparative example, only bone implantation was performed on the cervical vertebrae of 5 immunodeficient model rats, and bone healing was evaluated in the same manner.

As a result, in the group of combination with nonwoven fabric in which the sheet with lamination of the PCL nonwoven fabric and the chitin nonwoven fabric was used in combination, bone healing was made in 3 out of 3 rats, that is, the bone healing rate was 100%. On the other hand, in the group of only bone implantation of the comparative example, bone healing was made in only 1 out of 5 rats, that is, the bone healing rate was 20%. Further, the bone healing was evaluated by CT images at six weeks after the bone implantation, and this revealed that, as illustrated in FIG. 7, there were gaps between bones and sufficient bone healing was not made in the group of only bone implantation. However, it was confirmed that sufficient bone healing was made in the group of combination with nonwoven fabric.

The bone healing was evaluated by immunostaining images at six weeks after the bone implantation, and it was confirmed that the human MSCs were engrafted in the rat bodies in the group of combination with PCL nonwoven fabric. To be specific, cells positive for both human nuclear antigen (HNA) and DAPI are human mesenchymal stem cells, and cells positive only for DAPI are cells of the rat. As illustrated in FIG. 8, when the HNA image on the left and the DAPI image in the middle are superimposed on each other, the image on the right is obtained. When this image was observed, there were many cells positive for both HNA and DAPI. Thus, in the group of combination with PCL nonwoven fabric, it was confirmed that bone healing occurred even in view of the cellular level.

This application is based on Japanese Patent Application No. 2022-100731 filed on June 22, 2022 and Japanese Patent Application No. 2023-54932 filed on March 30, 2023 and includes the specifications, claims, drawings, and abstracts thereof. The disclosures in the above Japanese Patent applications are incorporated herein by reference in their entireties.

### [Industrial Applicability]

The tissue regeneration promoting sheet of the present invention can stably retain stem cells and therefore is useful.

### [List of Reference Symbols]

- 10: tissue regeneration promoting sheet
- 12: cell retention sheet
- 12A: first cell retention sheet
- 12B: second cell retention sheet
- 14: reinforcement sheet
- 20: holder

## Claims

1. A tissue regeneration promoting sheet comprising a nonwoven fabric, the nonwoven fabric containing fibers based on polycaprolactone as a main component, and the nonwoven fabric being configured to be impregnated with a cell suspension solution containing stem cells.

2. The tissue regeneration promoting sheet according to claim 1, wherein the fiber diameter of the fibers forming the nonwoven fabric is in a range of 300 nm to 2 µm.

3. The tissue regeneration promoting sheet according to claim 1 or 2, wherein the basis weight of the nonwoven fabric is in a range of 1 g/m² to 3 g/m².

4. The tissue regeneration promoting sheet according to any one of claims 1 to 3, wherein the thickness of the nonwoven fabric is larger than or equal to 300 µm.

5. The tissue regeneration promoting sheet according to any one of claims 1 to 4 further comprising a reinforcement sheet laminated on the nonwoven fabric and configured to be impregnated with the cell suspension solution.

6. The tissue regeneration promoting sheet according to claim 5, wherein the reinforcement sheet is another nonwoven fabric formed of fibers that are thicker than the fibers forming the nonwoven fabric.

7. The tissue regeneration promoting sheet according to any one of claims 1 to 6, wherein the nonwoven fabric comprises a first nonwoven fabric and a second nonwoven fabric, the second nonwoven fabric being laminated on the first nonwoven fabric and being less likely to be deformed than the first nonwoven fabric.
